# EUROPEAN PATENT SPECIFICATION

(11) **EP 2 059 301 B1**
(45) Date of publication and mention of the grant of the patent: **02.05.2012**
(21) Application number: 07837935.1
(22) Date of filing: 07.09.2007
(51) Int. Cl.: A61N 1/39, A61N 1/375

(54) **SYSTEM COMPRISING HERMETICALLY SEALED BODIES AND METHOD OF STORING AN IMPLANTABLE DEVICE**
SYSTEM MIT HERMETISCH VERSCHLOSSENEN KÖRPERN UND VERFAHREN ZUR AUFBEWAHRUNG EINER IMPLANTIERBAREN VORRICHTUNG
PROCÉDÉ ET DISPOSITIF DE CLÉ DYNAMOMÉTRIQUE BIDIRECTIONNELLE

(30) Priority: 08.09.2006 US 825052 P
(43) Date of publication of application: 20.05.2009
(60) Divisional application: 11191116.0
(73) Proprietor: Cardiac Pacemakers, Inc., St. Paul MN 55112-5798 (US)
(72) Inventor: FRULAND, Benjamin, R., Plymouth, MN 55441 (US); PERSUITTI, Kevin, J., Andover, MN 55304 (US); DAHL, Scott, Minneapolis, MN 55418 (US)
(74) Representative: Vossius & Partner
(86) International application number: PCT/US2007/019603
(87) International publication number: WO 2008/030593

(56) References cited:
- EP-A- 0 052 690
- US-A- 4 037 277
- US-A- 4 262 673
- US-A- 4 461 194
- US-A- 4 479 489
- US-A- 4 832 021
- US-A- 5 509 928
- US-A- 6 128 984
- US-A1- 2004 215 282

## Description

### TECHNICAL FIELD

The invention relates to a system for fastening a male terminal with a driver according to the preamble of claim 1 and a method for storing an implantable device using this system.

Such a system and such a method is known from US 2004/0215282.

### BACKGROUND

There is an ongoing need to provide devices which can be exposed to fluids without malfunctioning. Some designs cannot simply be encased in a fluid compatible, sealed material, as they must be manipulated in use. Such is the case with some devices which are implanted in a person. Some implantable systems include interchangeable components which conduct electricity. For example, some designs use a set fastener to pinch a lead. Devices such as these must provide reliable electrical and mechanical connection which does not degrade in use.

Various problems are associated with such designs. Bodily fluids can reduce the operability of these devices by binding components to one another. As such, workers have attempted to provide covers for these devices. However, many covers which provide good sealing characteristics, such as silicone covers, present a problem of bonding to other components, or to themselves, in use and in storage.

An additional problem is that a fastener can become misaligned, or skew to their mating feature, during surgery. Further, some fastener designs encourage cross threading, especially when a doctor is inserting a driver through a seal. When inserting a driver through a seal, it is important to have the fastener in alignment with the seal opening; some designs do not encourage this. An additional problem is that some fastener designs can crush and damage the leads they pinch.

To solve the above objects there is provided, according to the invention, a system for fastening a male terminal with a driver according to claim 1 and a method for storing an implantable device using this system, according to claim 5.

One embodiment of the present subject matter includes a system which includes a device including a first body which is hermetically sealed, a first terminal coupled to the first body, a second body which is part of the device and which is hermetically sealed, a second terminal coupled to the second body, a threaded fastener coupling the first terminal and the second terminal and a torque wrench matable to the threaded fastener, the torque wrench including a tightening clutch and a loosening clutch, the tightening clutch adapted to slip at a first torque, the loosening clutch adapted to slip at a second torque other than the first torque.

Another embodiment is contemplated which provides a method which includes coupling a female terminal and a male terminal to connect an implantable lead to a body of an implantable device, pinching the male terminal against a surface of the body which is inside a recess defined by the female terminal by torquing, with a torque wrench, a fastener to a first torque and unpinching the male terminal by untorquing the fastener at a second torque, other than the first torque, which is less than a torque large enough to cause inelastic deformation of the torque wrench.

One embodiment of the present subject matter includes a system which includes a device including a first body which is hermetically sealed, a first terminal coupled to the first body, a second body which is part of the device and which is hermetically sealed, a second terminal coupled to the second body, means for removably coupling the first terminal and the second terminal by applying a torque; and means for torquing at a first torque, and untorquing at a second torque other than the first torque, the means for removably coupling the first terminal and the second terminal by applying a torque.

The present subject matter includes optional features in additional embodiments. Some of those embodiments include a fastener which is part of a cardiac rhythm management device, such as a pacemaker or a defibrillator. Some embodiments include a silicone cover. Some embodiments include a fastener having a convex distal portion. Some embodiments include a fastener mateable to a male hex driver. Embodiments include taking a device out of storage and inserting a male terminal into the device, and securing that terminal to the device using a fastener of the present subject matter. Openings in covers of the present subject matter are lubricated, in various embodiments. Other options are disclosed herein.

One embodiment of the present subject matter includes a system for fastening a male terminal with a driver. The embodiment includes a body defining an at least partially threaded recess which intersects with a female recess adapted to receive the male terminal. The embodiment includes a fastener having an at least partially threaded body and a flanged head adapted to mate with the driver, the fastener disposed at least part of the way in the first recess, the flange having a diameter which is larger than a diameter of the at least partially threaded recess. The embodiment includes a flexible septum or cover coupled to the body and covering the first recess, the flexible septum having an opening sized for passage of the driver. In the embodiment, a first mode of operation, the system is adapted to clamp the male terminal between the body and the fastener, with the opening closed and sealed, and in a second mode or storage mode of operation the system is adapted to not clamp the male terminal between the body and the fastener while the flange abuts the flexible septum such that the opening is open and unsealed.

Another embodiment is contemplated which provides a method storing an implantable device. The embodiments include assembling a fastener into a recess in a body of the implantable device, the recess being covered by a flexible septum having a slit. The embodiment further includes opening the slit by adjusting the fastener with respect to the device such that a flange of the fastener abuts the flexible septum. The embodiment includes packaging the implantable device for storage. The embodiment further includes storing the implantable device.

One embodiment of the present subject matter includes body means for receiving a fastener and a male terminal. The embodiment includes fastener means for securing the male terminal to the body means, for limiting the travel of the fastener into the body means, and for preserving alignment of the fastener means with respect to the body means when the male terminal is not secured to the body means. The embodiment further includes cover means for covering the fastener and for sealing the fastener means and the body means.

The present subject matter includes optional features in additional embodiments. Some of those embodiments include a fastener which is part of a cardiac rhythm management device, such as a pacemaker or a defibrillator. Some embodiments include a silicone cover. Some embodiments include a fastener having a convex distal portion. Some embodiments include a fastener mateable to a male hex driver. Embodiments include taking a device out of storage and inserting a male terminal into the device, and securing that terminal to the device using a fastener of the present subject matter. Openings in covers of the present subject matter are lubricated, in various embodiments. Other options are disclosed herein.

Further details about the present subject matter are found in the detailed description and appended claims. Other aspects will be apparent to persons skilled in the art upon reading and understanding the following detailed description and viewing the drawings that form a part thereof, each of which are not to be taken in a limiting sense. The scope of the present invention is defined by the appended claims.

The following items are preferred embodiments of the invention.

### BRIEF DESCRIPTION OF THE DRAWINGS

FIG. 1A illustrates a cross section of a fastener and a lead in an unfastened position.
FIG. 1B illustrates a cross section of a fastener and a lead in a fastened position.
FIG. 2 illustrates a cross section of a fastener and a lead in a lead unfastened position.
FIG. 3A illustrates a partial side view of a cross section of a fastener in an unfastened position, according to the present invention.
FIG. 3B illustrates a top view of the view in FIG. 3A.
FIG 4 illustrates a partial perspective view of a fastener.
FIG. 5 illustrates a partial cross section of a fastener.
FIG. 6 illustrates a torque wrench.
FIG. 7 illustrates a system including a torque wrench, a fastener, and terminals.
FIG. 8 shows a method of connecting a lead and a body.

### DETAILED DESCRIPTION

The following detailed description of the present subject matter refers to subject matter in the accompanying drawings which show, by way of illustration, specific aspects and embodiments in which the present subject matter may be practiced. These embodiments are described in sufficient detail to enable those skilled in the art to practice the present subject matter. References to "an", "one", or "various" embodiments in this disclosure are not necessarily to the same embodiment, and such references contemplate more than one embodiment. The following detailed description is demonstrative and not to be taken in a limiting sense. The scope of the present invention is defined by the appended claims.

The present invention provides a system for fastening a male terminal of one component of a system to another component of a system. There is provided a first terminal for connection of an implantable lead to a second terminal which is part of an implantable device. Devices contemplated include, but are not limited to, cardiac rhythm management electronics including, but not limited to, pacemakers, defibrillators, cardioverter defibrillators. The present subject matter additionally contemplates other devices which deliver an electrical pulse to the body. Additional devices beyond these also benefit from features disclosed herein. In the embodiments, the terminal for the lead is male. A fastener is threaded into a device such that the fastener may encroach into a recess of a female terminal of a device. As the fastener is tightened, it pinches against a male terminal inserted into the female terminal.

The present subject matter covers a range of fastener embodiments. In the embodiments the fastener includes threads. Additional embodiments not falling under the scope of the claims include fasteners which include a shaft with grooves which are adapted to mate with pins in a recess such that rotation of the fastener with respect to the recess receives the pins in the grooves and draws the fastener into the recess. Some of these embodiments limit the radial motion of the fastener. For example, fasteners contemplated go from being uncoupled to a recess at 0 degrees rotation, to fully coupled with a recess at 270 degrees rotation. Some of these embodiments provide tactile feedback so a fastener operator can feel when the fastener is fully engaged. Some embodiments provide features in the grooves of the fastener, such as ribs, which provide additional feedback. Such features additionally may discourage disengagement of the fastener from the recess.

There is provided a cover or septum which covers the fastener. In some of these embodiments, the septum provides at least a partial seal against bodily fluids. The septum includes an opening through which a driver can be inserted to adjust the fastener.

There is provided a fastener with a flanged head such that the fastener cannot be fastened into a fastener hole beyond a predetermined depth. These embodiments work to reduce damage to a male terminal during tightening of the fastener. The flange serves additional functions as well. Some of these are discussed herein.

Some embodiments provide a fastener having an unthreaded portion which extends away from the threads and leads the fastener into a mating fastener hole. These embodiments reduce instances of the fastener tipping, or become skew with respect to its mating recess, after it is disengaged from its recess. These embodiments additionally reduce instances of a fastener going out of alignment with an access opening in a cover which covers the fastener.

In various embodiments, materials which bond to themselves or which bond to other objects over time are provided. For example, a silicone cover is provided with an opening in it for accessing a fastener of the present subject matter. The fastener helps to keep an opening of a cover open so that the opening does not stick close or bond closed. The fastener keeps an opening of the cover open in storage. According to the invention the flanged head of the fastener keeps the opening open. In some embodiment, the opening is a slit, and is shaped to work in conjunction with the fastener to open when the fastener is backed out of the recess.

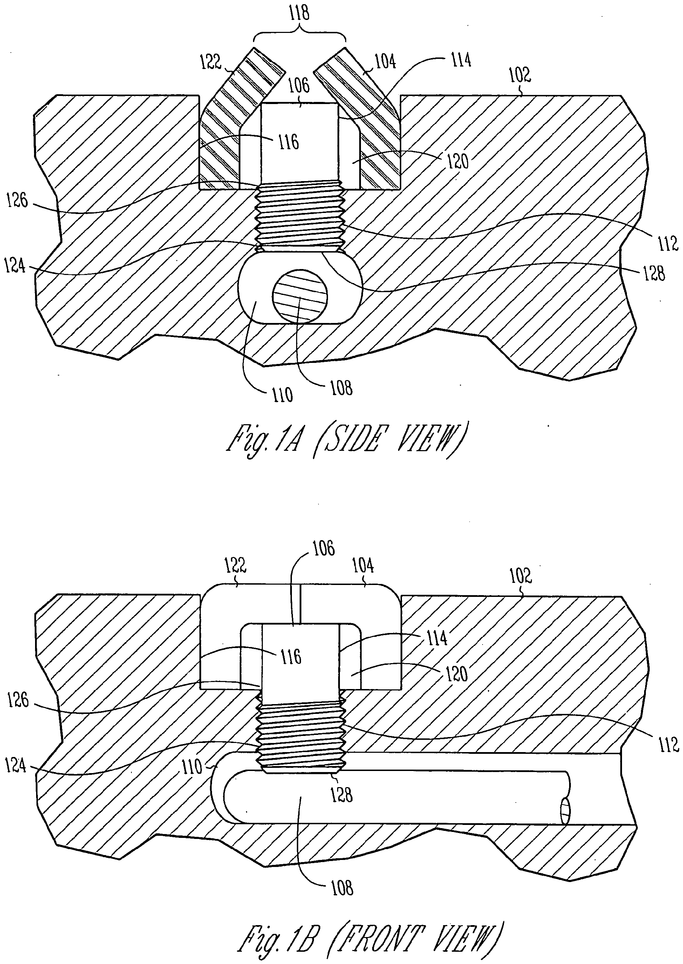

FIG. 1A illustrates a cross section of a fastener and a lead in an unfastened position, according to one embodiment not according to the present invention. FIG. 1B illustrates a cross section of a fastener and a lead in a fastened position, according to one embodiment not according to the present invention. The illustrated fastener 106 is a set screw.

Various embodiments provide a system for fastening a fastener 106 with a driver. Drivers include a range of interfaces. Interfaces include, but are not limited to, hex socket, hex head, TORX, slot, PHILLIPS, and/or combinations thereof. This list is not exhaustive or exclusive of the present subject matter, and additional fasteners designs are also contemplated. TORX is a registered trademark of Textron, Inc., which is incorporated in Delaware and has an office at 40 Westminster Street in Providence, Rhode Island 02903. PHILLIPS is a registered trademark of Phillips Screw Company, which is incorporated in Delaware and has an office at 8 Mercer Road in Natick, Massachusetts 01760. In some embodiments , the driver provides the male interface, and the fastener provides the female interface. Additional embodiments are contemplated in which the driver includes the female interface, and the fastener includes the male interface. Additional drives which engage fasteners using techniques not expressly disclosed here are also contemplated.

The present subject matter provides a body 102 defining an at least partially threaded recess 112 for a fastener. In various embodiments, the recess 112 includes a distal portion 124 and a proximal portion 126. Bodies include housings for implantable devices, as well as feedthroughs or headers which are attached to one or more additional devices. The embodiments provide a body 102 which seals its contents. For example, some bodies seal housed electronics from the encroachment of bodily fluids.

The recess intersects with a female recess 110 adapted to receive the male terminal 108. Various male terminals are contemplated by the present subject matter, including male terminals for leads. Male terminals for sensors and for stimulation electrodes which are not on a lead are also contemplated. Other components having a male terminal are additionally compatible with the present subject matter.

The male terminal 108 is inserted into the female terminal, and is pinched. Pinching is performed by twisting a driver coupled to the fastener. In various embodiments, the driver is torque limited. In additional embodiments, the driver is not torque limited.

The present subject matter includes a cover 104 or flexible septum for the fastener 106. In some embodiments, the cover 104 is integrated with the body 102. Some of these embodiments provide a polymer body 102 and cover 104. This configuration is one embodiment contemplated by the present subject matter. Additional embodiments, in which the cover 104 is not integrated with the body 102 are contemplated as well.

In the embodiments of figures 1A and 1B, the fastener 106 includes a proximal portion 114 and a distal portion 128. A recess 116 is an opening countersunk into body 102. The recess 116 is cylindrical, but other shapes are possible. In embodiments having such a recess, various embodiments include a cover 104 which is fit into recess 116. In various embodiments, the cover 104 is a plug which is interference fit into recess 116. In various embodiments, the interference fit includes additional features, such as ribs, threads, teeth, or other fastening features. The fit of cover 104 into recess 116 is such that the fastener 106 can open an opening 118 in cover 104 without unseating the cover 104 from the recess 116.

The cover 104 defines a septum which defines a chamber 120 under which fastener 106 is disposed. Such a relationship, in various embodiments, reduces instances of encroachment of fluids into chamber 120. Although the pictured embodiment includes a chamber 120 which is larger than the fastener 106, the present subject matter is not so limited and includes chambers which are form fitting to the fastener. Such designs can reduce the size of a device. In implanted embodiments, this can result in improved patient comfort. In various embodiments, this can also result in easier implantation procedures for care givers.

According to the invention the septum is flexible. In various embodiments, the septum includes silicone. In additional embodiments, the septum is another polymer, or a combination of another polymer and silicone. This is not an exhaustive or exclusive list, and additional materials are contemplated.

The septum includes an opening sized for passage of a driver. Some embodiments of the present subject matter include a septum having an opening sized for passage of a hex head driver mateable to the fastener 106. The opening is sized such that the driver may pass through the opening without tearing or otherwise harming the opening.

There is provided a first mode of operation as depicted in FIG. 1B. In that mode, a system clamps the male terminal between the body and the fastener, with the opening closed and sealed. Some sealed embodiments provide a seal which resists flow of bodily fluids. Additional seals do not resist the flow of bodily fluids, but otherwise provide a seal. Examples of such seals are seals which keep out dust.

There is provided a second mode of operation as depicted in FIG. 1A. The second mode includes a system which does not clamp the male terminal between the body and the fastener. This second mode, includes a fastener 106 which abuts the septum 122 such that the opening is open and unsealed. In various embodiments, the opening 118 is shaped like a slit in the cover 104. The present subject matter includes openings having additional shapes as well, such as circular holes adapted to seal unto themselves.

In various embodiments, the fastener 106 is radioopaque. In some of these embodiments, care givers are able to better determine the orientation of an implantable device with respect to a patient. An additional benefit is that the engagement of the male terminal with the female terminal can be studied without explant. This is not an exhaustive or exclusive list of benefits provided.

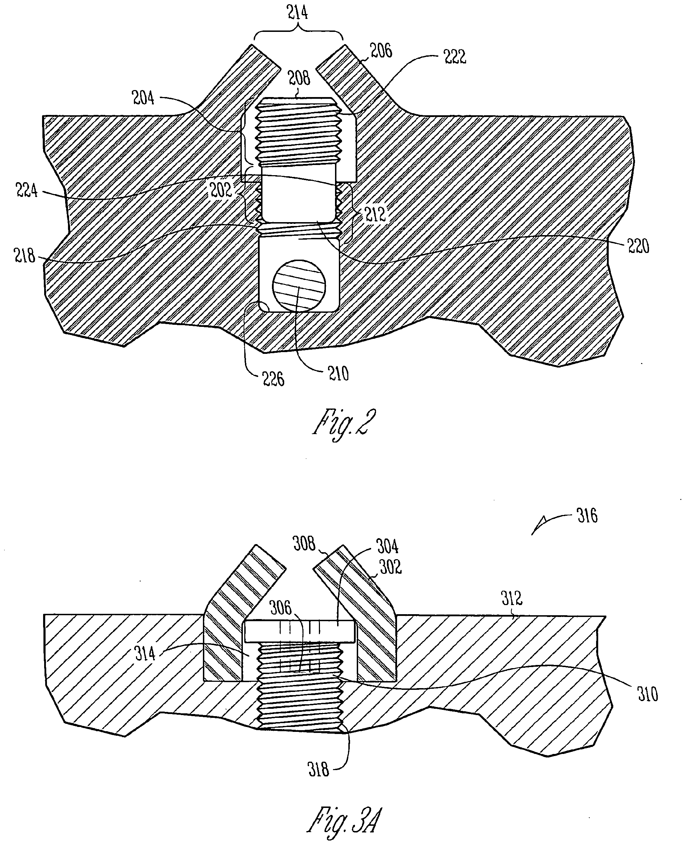

FIG. 2 illustrates a cross section of a fastener 208 and a lead 210 in a lead unfastened position, according to one embodiment not falling under the scope of the present invention. A cover 206 is provided. In the illustration, the cover is flexed such that an opening 214 is demonstrated. The opening 214 is large enough for the fastener 208 to pass through. In various embodiments, a driver which can couple with the fastener and apply a torque to the fastener is contemplated.

The fastener 208 illustrated is one of a range which are partially threaded. The fastener includes a proximal end 222 and a distal end 220. The fastener includes a threaded portion 204 and an unthreaded portion 202.The fastener is partially engaged with the recess 218. The recess 218 includes a proximal end 224 and a distal end 226. The unthreaded portion 202 extends into the recess and at least partially engages the recess 218. One or more threads of the fastener 208 may engage one or more threads of the recess 218.

When the threaded portion of the fastener 208 is not fully threaded into the recess 218, a device operator is extracting fastener 208 from the recess 218. An operator may seek to perform such an operation for various reasons. One reason is that a care giver might want to explant a first device to replace it with a second device. The care giver may want to mate the lead of the first device to the second device. Other reasons are additionally contemplated. In these situations, the care giver backs the fastener 208 out of the recess 218 using multiple turns with a driver. In some of these embodiments, the care giver backs the fastener 208 out of the recess 218 until no threads of the fastener 208 engage threads of the recess 218. In embodiments which do not include a threaded portion 212 of the fastener 208 and therefore do not fall under the scope of the claims, the fastener 208 might tip or otherwise go out of alignment with the recess 218, making reinsertion of the fastener 208 into the recess 218 more difficult. Embodiments of the present subject matter address this issue. Embodiments may additionally address issues not recited herein expressly.

Some embodiments of the present subject matter reduce the need to include features in recess 218 which stop the progress of fastener 208 as it is positioned within recess 218. For example, some embodiments of the present subject matter which do not include unthreaded portion 220 can tip upon extraction from the recess 218. As such, in some embodiments, a fastener is fully disposed in a recess. A stopper is then inserted near the proximal end 224 of the recess such that the fastener cannot be removed easily from the recess 218. One example of a stopper is a washer welded into recess 218, but other embodiments are contemplated. These embodiments present an issue when an operator uses excessive torque in an attempt to overcome the stopper without realizing that the stopper is impeding the progress of the extraction of the fastener from the recess. The issue is that the stopper and the fastener can become wedged together. To reduce instances of wedging, there is provided an unthreaded portion 212 to reduce instances of tipping.

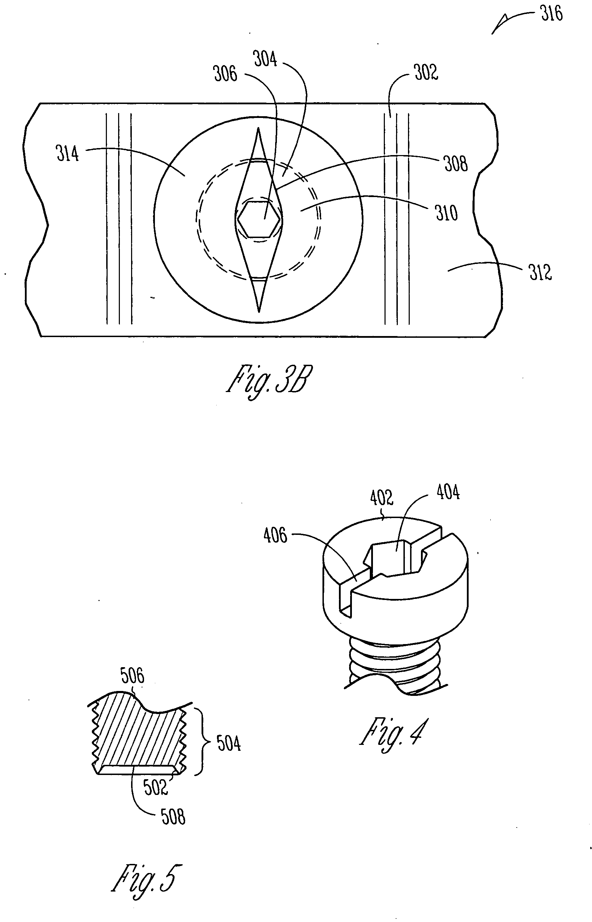

FIG. 3A illustrates a partial side view of a cross section of a fastener in an unfastened position, according to one embodiment of the present invention FIG. 3B illustrates a top view of the view in FIG. 3A. The embodiment includes a fastener 310 which is fastened to body 312. The fastener includes a flanged portion 304. The fastener additionally includes a recess 306 mateable to a driver. In additional embodiments, the fastener is a hex head fastener. Embodiments are contemplated in which the fastener 310 does not include a recess 306 and is actuated another way.

The embodiment includes a cover or flexible septum 302. In some of these embodiments, the cover 302 has a slit 308. The slit is closeable such that it seals a chamber 314. In some embodiments, the chamber 314 is sealed from the encroachment of bodily fluids into the chamber 314.

In some embodiments, the cover 302 is manufactured from silicone. Over prolonged periods of time, two pieces of silicone can partially or fully bond together when put into contact with one another. As such, the present subject matter provides an operator of the fastener 310 the option to store device 316 with the fastener configured as illustrated in FIG. 3A. The fastener includes a flange 304 which is oversized, having a large diameter than the diameter of the recess 318. This allows for various functions. One function is to hold open an opening in the cover 302 so that silicone does not bond with itself. Another function is to limit the travel of the fastener 310 into the recess 318. Such a limit can protect against crushing of a male terminal which the fastener 310 cinches against the body 312. If a fastener system can protect against crushing, device 316 sellers do not have to package the device with a torque limited driver. Further, reducing instances of crushing can reduce the need to train operators on how to avoid crushing a terminal while operating fastener 310. Another benefit of the present subject matter is that the opening in the cover 302 provides a visual cue that the fastener 310 is not yet inserted into the recess 318.

FIG 4 illustrates a partial perspective view of a fastener . The fastener 402, in various embodiments, includes a recess 404. In some of these embodiments, the recess 404 is a hex socket, as pictured. Embodiments of the present subject matter additionally include a slot 406, either alone, or in combination with another feature suited for coupling fastener 402 with a driver. Embodiments may additionally include other features as disclosed herein.

FIG. 5 illustrates a partial cross section of a fastener. Illustrated is a fastener 506. The illustrated cross section is the distal portion of a fastener 506. The fastener includes a threaded portion 504. In various embodiments, the distal end of the fastener 506 is substantially convex. In some embodiments, the substantially convex feature includes a substantially planar portion 508 and a bevel 502. Additional shapes are possible, however, including a partial sphere shape, in some embodiments.

The present invention includes a method for storing an implantable device. The method includes assembling a fastener into a recess in a body of the implantable device. The recess is covered. According to the invention the recess is covered by a flexible septum.

The septum has an opening. In some of these embodiments, the opening is a slit. The slit may be opened by adjusting the fastener with respect to the device. The fastener backs into the septum and forces the slit open. The fastener includes a flange which aids in opening the slit. The flange of the fastener abuts the flexible septum. The flange additionally prevents overtorquing of the fastening during tightening.

The embodiment includes packaging the implantable device for storage and storing the implantable device. Storage practices contemplated include, but are not limited to, warehousing, storing with a reseller, storing with a sales person, and other storing practices not recited herein expressly.

An additional process includes removing the device from storage, inserting a male terminal into the body of the implantable medical device and coupling the male terminal to the body of the device by tightening the fastener against the male terminal.

Additional processes include applying a lubricant to the slit. Examples of lubricants contemplated by the present subject matter include, but are not limited to, MDX4-4159, NuSil MED420, MED-4159, MED-4162, MED1-4162, and/or NUSIL, and combinations thereof. NUSIL is a registered trademark of NUSIL TECHNOLOGY headquartered in Carpinteria, CA, USA. MDX4-4159, NuSil MED420, MED-4159, MED-4162, MED1-4162 each are manufactured by DOW CORNING, which is headquartered in Midland, MI, USA.

Additional embodiments contemplate bundling the implantable device with a torque limited driver for adjusting the fastener. In various embodiments, the torque driver is torque limited during tightening. In additional embodiments, the torque driver is torque limited during loosening. Torque drivers which are torque limited during tightening and loosening are additionally contemplated by some embodiments. Embodiments are contemplated in which a non-torque limited driver is bundled with a device of the present subject matter.

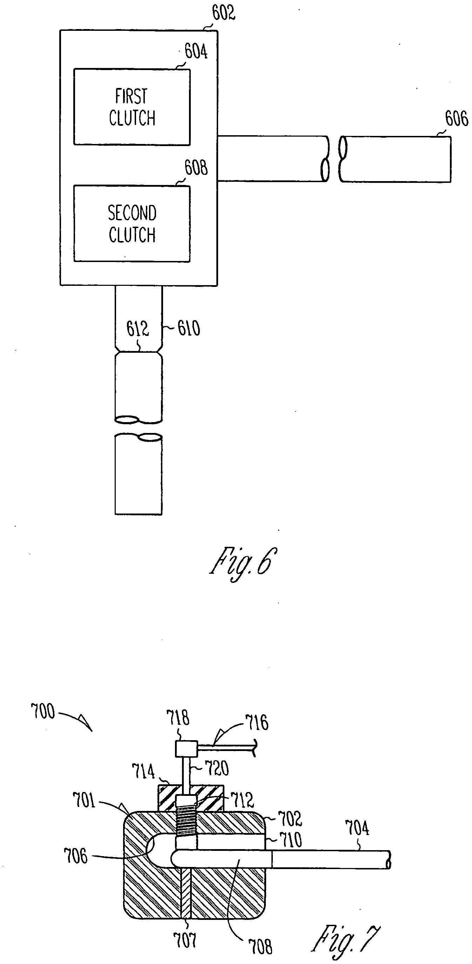

FIG. 6 illustrates a torque wrench 600. Some embodiments include a torque wrench having head 602 which includes a first clutch 604. In various embodiments, a handle 606 is included to provide mechanical advantage. The first clutch 604 provides a specified amount of torque during torquing and slips when that torque is exceeded. The specified torque can be reached during tightening only, during loosening only, or during both tightening and loosening. Some embodiments which provide for slipping at a specified torque during both tightening and loosening include a switch to toggle between tightening mode and loosening mode. Additional embodiments do not require a switch to switch between tightening mode and loosening mode.

Various embodiments click when the specified torque is reached. Some embodiments do not click when the specified torque is reached. Some embodiments provide tactile feedback when a specified torque is reached.

In some examples, the specified torque is predetermined and a care provider cannot adjust the torque. For example, some embodiments include a first clutch 604 which slips at a specified torque, and which is not adjustable without remanufacturing the clutch.

Some embodiments include a second clutch 608 which slips at a different torque than the first clutch 604 slips at. Such embodiments can provide a first torque during tightening, and a second torque during loosening. The second clutch can be adjustable or nonadjustable, and can optionally provide feedback (e.g., audible, tactile) if desired. As such, some examples provide a torque wrench including a tightening clutch and a loosening clutch, the tightening clutch adapted to slip at a first torque, the loosening clutch adapted to slip at a second torque other than the first torque.

In various embodiments, the torque wrench 600 includes a driver 610. The driver can be any of the drivers discussed here, including hex drivers. In various embodiments, the driver includes a stress riser 612. The stress riser, in various embodiments, is intended to facilitate a controlled failure of the driver 610 under torque. For example, in some embodiments, first clutch slips as a first torque (e.g., around 15 inch pounds during tightening), second clutch slips at a second torque (e.g., around 22 inch pounds during loosening), and the stress riser causes a controlled break of driver 610 at a torque above the second torque. Such a design reduces instances of debris in a wound during driver 610 breakage. In various embodiments, the stress riser 612 is the result of scoring. Scoring can include cast features, ground features, excised features, pressed features, and other features which result in a stress riser.

In various embodiments, tightening clutch does not include lubricant. In additional embodiments, the clutch does not include a lubricant. Some embodiments incorporate a torque wrench which includes plastic, such as a plastic handle or one or more plastic clutch housing. Some embodiments include a tightening clutch which is plastic. Additional embodiments include a loosening clutch which is plastic.

FIG. 7 illustrates a system 700 including a torque wrench, a fastener, and terminals. Illustrated is a device 701 which includes a first body 702 and a second body 704. The first body 702 and the second body 704 are implantable. One or both of the first body 702 and the second body 704 are hermetically sealed. There is included a first terminal 706. The first terminal includes a physical component which couples the first terminal 706 to the first body 702. According to the invention the first terminal 706 is a recess defined in part by the first body 702. Such a configuration provides a female terminal. Other configurations not falling under the scope the claims are possible, including embodiments which the first terminal 706 is not a female terminal.

Additionally provided is a second terminal 708. The second terminal 708 is coupled to the second body 704. The illustrated embodiment provides a second body 704 and a second terminal 708 which is mateable to a recess, such as the first terminal 706. Such a configuration provides a male terminal. A conductor 707 is included in the first terminal 706. The second terminal 708 additionally includes conductor (e.g., a metallic coating) so that, when connected, the first terminal 706 can conduct electrical current with the second terminal 708. The illustrated conductor 707 includes metal, and the illustrated second terminal 708 includes at least a metal coating. In various embodiments, the first terminal 706 is integrated with the conductor, such as by potting or by another method. In some embodiments, the entire first terminal includes conductor. Other conductor configurations are possible, including male/female parings for the conductors, and other conductor connection configurations. Some embodiments include a seal 710 which seals one or more conductors from the ambient atmosphere of system 700.

There is included a threaded fastener 712. The threaded fastener illustrated is disposed at least partially across the female terminal 706. The threaded fastener is disposed in an at least partially threaded recess adapted to receive the fastener. The first body 702 defines a recess to receive the fastener 712. The fastener 712 couples the male terminal and the female terminal. The fastener 712 has an at least partially threaded body and a flanged head adapted to mate with the torque wrench 716, the fastener disposed at least part of the way in the female recess (e.g., the first terminal 706), the flange having a diameter which is larger than a diameter of an at least partially threaded recess adapted to receive the fastener.

The embodiment includes a seal 714 which seals the fastener and the recess in which the fastener is disposed. In some embodiments, the seal 714 seals the fastener 712 from exposure to the atmosphere of system 700. The seal 714 includes a flexible septum coupled to the first body 702 and covering the at least partially threaded recess, the flexible septum having an opening sized for passage of the torque wrench. In various embodiments, an opening includes a slit. In some embodiments, the flexible septum includes silicone.

Various embodiments include a torque wrench 716 which is matable to the threaded fastener 712. In various embodiments, the torque wrench includes a head 718 which includes at least one clutch (e.g., a tightening clutch and a loosening clutch). The torque wrench 716 includes a driver 720 in various embodiments. Various embodiments include a wrench that is mateable to the threaded fastener and that does not include a clutch. A standard L-shape or T-shaped style hex wrench is provided in various embodiments. Some of these embodiments include a stress riser.

In a first mode of operation, the male terminal 704 is clamped between the first body 702 and the fastener 712. In a second mode of operation, the male terminal 704 is not clamped between the first body 702 and the fastener 712. The septum functions as do septums mentioned in other discussions in this document.

In various embodiments, the first body 712 includes a case including pulse generation electronics. Titanium cases are contemplated in some embodiments. Cases which are hermetically sealed are also contemplated. In various embodiments, the pulse generation electronics include pacemaker electronics. In additional embodiments, the pulse generation electronics include defibrillation electronics.

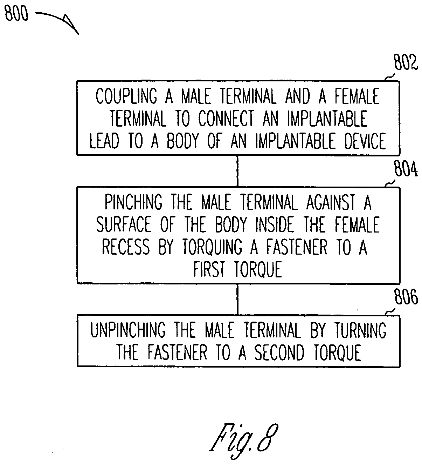

FIG. 8 shows a method 800 of connecting a lead and a body. At 802, there is included coupling a female terminal and a male terminal to connect an implantable lead to a body of an implantable device. At 804, there is included pinching the male terminal against a surface of the body inside the female recess by torquing a fastener to a first torque. In various embodiments, a torque wrench is used to apply the torque. At 806, there is included unpinching the male terminal by turning the fastener to a second torque. In some of these embodiments, the second torque is less than a torque large enough to cause inelastic deformation of the torque wrench. A method may include untorquing the fastener with a second wrench at a torque which is greater than the second torque. A method may include delivering electrical pulses from the implantable device to an electrode of the implantable lead over a conduction path through the male terminal and the female terminal.

Some embodiments include a torque wrench with a driver. In some of these embodiments, the driver is scored. As such, various embodiments include scoring a driver of the torque wrench to define a stress riser. Various additional embodiments include inserting a driver of the torque wrench through a flexible septum coupled to the body and covering an at least partially threaded recess in which the fastener is disposed, the flexible septum having an opening sized for passage of the driver.

## Claims

1. A system for fastening a male terminal (108; 704) with a driver (610; 720), the system comprising:
a body (312) defining an at least partially threaded recess (306) which intersects with a female recess adapted to receive the male terminal;
a fastener (310; 402; 506) having an at least partially threaded portion (204) and a flanged head (304) adapted to mate with the driver (610), the fastener (310; 402; 506) disposed at least part of the way in the at least partially threaded recess, the flange (304) having a diameter which is larger than a diameter of the at least partially threaded recess (306); and
a flexible septum (302) coupled to the body (312) and covering the at least partially threaded recess (306), the flexible septum (302) having an opening sized for passage of the driver (610); **characterized in that** the system is configured such that
the fastener is adjustable, with respect to the body, between a first mode of operation, in which the system is adapted to clamp the male terminal between the body (312) and the fastener (310; 402; 506), with the flange (304) disposed against the body (312), limiting travel of the fastener into the male terminal, and with the opening closed and sealed, and a storage mode in which the male terminal is slidable into the body (312) while the fastener (310) is at least partially threaded into the at least partially threaded recess (306) and the flange (304) abuts the flexible septum (302) such that the opening is open and unsealed to receive the driver.

2. The system of claim 1, wherein the opening is a slit (308).

3. The system of claim 1, wherein the fastener (310; 402; 506) has an unthreaded fastener portion disposed near the distal portion of the fastener (310; 402; 506), the fastener (310; 402; 506) having an overall length such that in the second mode of operation, a threaded fastener portion is disposed at least partially in the at least partially threaded recess (306).

4. The system of claim 1, wherein the body (312) is part of an implantable, device (316).

5. A method for storing an implantable device (316) using a system of any one of claims 1 to 3, the method comprising:
assembling a fastener (310; 402; 506) into a recess in a body (312) of the implantable device (316), the recess being covered by a flexible septum (302) having a slit (308),
opening the slit (308) by adjusting the fastener (310; 402; 506) with respect to the device (316) such that a flange of the fastener (310; 402; 506) abuts the flexible septum (302);
packaging the implantable device (316) for storage; and
storing the implantable device (316).

6. The method of claim 5, further comprising inserting a male terminal into the body (312) of a implantable device (316), and coupling the male terminal to the body (312) of the device (316) by tightening the fastener (310; 402; 506) against the male terminal.

7. The method of claim 5, further comprising applying a lubricant to the slit (308).

8. The method of claim 7, further comprising bundling the implantable device (316) with a torque limited driver (610) for adjusting the fastener (310; 402; 506).

9. The system of claim 1, wherein the flexible septum (302) includes silicone.

10. The system of claim 1, wherein the distal end of the fastener (310; 402; 506) is substantially convex (502; 508).

11. The system of claim 1, wherein the fastener (310; 402; 506) includes a hexagonal recess adapted to couple to the driver (610).

12. The system of claim 11, wherein the regular hexagonal recess includes a slot (406).

13. The system of claim 4, wherein the implantable, device (316) is a pacemaker.

14. The system of claim 4, wherein the implantable, device (316) is a cardioverter defibrillator.

15. The system of claim 2, further comprising a lubricant applied to the slit (308) of the flexible septum (302).

## Patentansprüche

1. System zum Befestigen eines Steckanschlusses (108; 704) mit einem Schraubwerkzeug (610; 720), wobei das System aufweist:
einen Körper (312), der eine mindestens teilweise mit einem Gewinde versehene Aussparung (306) bildet, die eine Buchsenaussparung schneidet, die geeignet ist, den Steckanschluss aufzunehmen;
ein Befestigungselement (310; 402; 506) mit einem mindestens teilweise mit einem Gewinde versehenen Abschnitt (204) und einem Flanschkopf (304), der geeignet ist, sich mit dem Schraubwerkzeug (610) zu paaren, wobei das Befestigungselement (310; 402; 506) über mindestens einen Teil des Wegs in der mindestens teilweise mit einem Gewinde versehenen Aussparung angeordnet ist und der Flansch (304) einen Durchmesser hat, der größer als ein Durchmesser der mindestens teilweise mit einem Gewinde versehenen Aussparung (306) ist; und
eine flexible Wand (302), die mit dem Körper (312) gekoppelt ist und die mindestens teilweise mit einem Gewinde versehene Aussparung (306) abdeckt, wobei die flexible Wand (302) eine Öffnung hat, die zum Durchgang des Schraubwerkzeugs (610) bemessen ist; **dadurch gekennzeichnet, dass** das System so konfiguriert ist, dass das Befestigungselement im Hinblick auf den Körper einstellbar ist zwischen einem ersten Betriebsmodus, in dem das System geeignet ist, den Steckanschluss zwischen dem Körper (312) und dem Befestigungselement (310; 402; 506) zu verspannen, wobei der Flansch (304) am Körper (312) angeordnet ist, was die Bewegung des Befestigungselements in den Steckanschluss begrenzt, und wobei die Öffnung geschlossen und abgedichtet ist, sowie einem Lagermodus, in dem der Steckanschluss in den Körper (312) verschiebbar ist, während das Befestigungselement (310) mindestens teilweise in die mindestens teilweise mit einem Gewinde versehene Aussparung (306) geschraubt ist und der Flansch (304) an der flexiblen Wand (302) anliegt, so dass die Öffnung offen und nicht abgedichtet ist, um das Schraubwerkzeug aufzunehmen.

2. System nach Anspruch 1, wobei die Öffnung ein Schlitz (308) ist.

3. System nach Anspruch 1, wobei das Befestigungselement (310; 402; 506) einen gewindelosen Befestigungselementabschnitt hat, der nahe dem distalen Abschnitt des Befestigungselements (310; 402; 506) angeordnet ist, wobei das Befestigungselement (310; 402; 506) eine solche Gesamtlänge hat, dass im zweiten Betriebsmodus ein mit einem Gewinde versehener Befestigungselementabschnitt mindestens teilweise in der mindestens teilweise mit einem Gewinde versehenen Aussparung (306) angeordnet ist.

4. System nach Anspruch 1, wobei der Körper (312) Teil einer implantierbaren Vorrichtung (316) ist.

5. Verfahren zum Lagern einer implantierbaren Vorrichtung (316), die ein System nach einem der Ansprüche 1 bis 3 verwendet, wobei das Verfahren aufweist:
Einbauen eines Befestigungselements (310; 402; 506) in eine Aussparung in einem Körper (312) der implantierbaren Vorrichtung (316), wobei die Aussparung durch eine flexible Wand (302) mit einem Schlitz (308) abgedeckt ist,
Öffnen des Schlitzes (308) durch Einstellen des Befestigungselements (310; 402; 506) im Hinblick auf die Vorrichtung (316), so dass ein Flansch des Befestigungselements (310; 402; 506) an der flexiblen Wand (302) anliegt;
Verpacken der implantierbaren Vorrichtung (316) zur Lagerung; und
Lagern der implantierbaren Vorrichtung (316).

6. Verfahren nach Anspruch 5, das ferner aufweist: Einsetzen eines Steckanschlusses in den Körper (312) einer implantierbaren Vorrichtung (316) und Koppeln des Steckanschlusses mit dem Körper (312) der Vorrichtung (316) durch Festziehen des Befestigungselements (310; 402; 506) am Steckanschluss.

7. Verfahren nach Anspruch 5, das ferner aufweist: Auftragen eines Gleitmittels auf den Schlitz (308).

8. Verfahren nach Anspruch 7, das ferner aufweist: Bündeln der implantierbaren Vorrichtung (316) mit einem drehmomentbegrenzten Schraubwerkzeug (610) zum Einstellen des Befestigungselements (310; 402; 506).

9. System nach Anspruch 1, wobei die flexible Wand (302) Silikon aufweist.

10. System nach Anspruch 1, wobei das distale Ende des Befestigungselements (310; 402; 506) im Wesentlichen konvex (502; 508) ist.

11. System nach Anspruch 1, wobei das Befestigungselement (310; 402; 506) eine Sechskantaussparung aufweist, die geeignet ist, sich mit dem Schraubwerkzeug (610) zu koppeln.

12. System nach Anspruch 11, wobei die regelmäßige Sechskantaussparung einen Schlitz (406) aufweist.

13. System nach Anspruch 4, wobei die implantierbare Vorrichtung (316) ein Schrittmacher ist.

14. System nach Anspruch 4, wobei die implantierbare Vorrichtung (316) ein Kardioverter/Defibrillator ist.

15. System nach Anspruch 2, ferner mit einem Gleitmittel, das auf den Schlitz (308) der flexiblen Wand (302) aufgetragen ist.

## Revendications

1. Système de fixation d'une connexion mâle (108 ; 704) au moyen d'un outil (610 ; 720), ledit système comprenant :
un corps (312) définissant une cavité (306) au moins partiellement filetée qui intersecte une cavité femelle prévue pour recevoir la connexion mâle ;
un élément de fixation (310 ; 402 ; 506) avec une partie (204) au moins partiellement filetée et une tête plate (304) prévue pour s'ajuster à l'outil (610), ledit élément de fixation (310 ; 402 ; 506) étant disposé au moins en partie dans la cavité au moins partiellement filetée, la tête plate (304) étant de diamètre supérieur au diamètre de la cavité (306) au moins partiellement filetée ; et
une cloison flexible (302) raccordée au corps (312) et recouvrant la cavité (306) au moins partiellement filetée, ladite cloison flexible (302) présentant une ouverture dimensionnée pour le passage de l'outil (610) ; **caractérisé en ce que** ledit système est configuré de manière à permettre un ajustement de l'élément de fixation par rapport au corps, entre un premier mode de service où le système est apte à serrer la connexion mâle entre le corps (312) et l'élément de fixation (310 ; 402 ; 506), avec la tête plate (304) disposée contre le corps (312), en limitant la pénétration de l'élément de fixation dans la connexion mâle, et avec l'ouverture obturée et hermétiquement scellée, et un mode de stockage où la connexion mâle est insérable dans le corps (312) cependant que l'élément de fixation (310) est au moins partiellement vissé dans la cavité (306) au moins partiellement filetée et que la tête plate (304) repose contre la cloison flexible (302) de manière à laisser l'ouverture ouverte et non scellée pour recevoir l'outil.

2. Système selon la revendication 1, où l'ouverture est une fente (308).

3. Système selon la revendication 1, où l'élément de fixation (310 ; 402 ; 506) présente une partie d'élément de fixation non filetée à proximité de la partie distale de l'élément de fixation (310 ; 402 ; 506), ledit élément de fixation (310 ; 402 ; 506) ayant une longueur totale telle que dans le deuxième mode de service, une partie filetée de l'élément de fixation est disposée au moins en partie dans la cavité (306) au moins partiellement filetée.

4. Système selon la revendication 1, où le corps (312) fait partie d'un dispositif (316) implantable.

5. Procédé de stockage d'un dispositif implantable (316) recourant à un système selon l'une quelconque des revendications 1 à 3, ledit procédé comprenant les étapes suivantes
montage d'un élément de fixation (310 ; 402 ; 506) dans une cavité d'un corps (312) du dispositif implantable (316), la cavité étant recouverte par une cloison flexible (302) pourvue d'une fente (308),
ouverture de la fente (308) par ajustement de l'élément de fixation (310 ; 402 ; 506) par rapport au dispositif (316) de manière à faire reposer une tête plate de l'élément de fixation (310 ; 402 ; 506) contre la cloison flexible (302) ;
emballage du dispositif implantable (316) pour stockage ; et
stockage du dispositif implantable (316).

6. Procédé selon la revendication 5, comprenant en outre les étapes d'insertion d'une connexion mâle dans le corps (312) d'un dispositif implantable (316), et de raccordement de la connexion mâle au corps (312) du dispositif (316) par serrage de l'élément de fixation (310 ; 402 ; 506) contre la connexion mâle.

7. Procédé selon la revendication 5, comprenant en outre l'étape d'application d'un lubrifiant sur la fente (308).

8. Procédé selon la revendication 7, comprenant en outre l'étape d'accouplement du dispositif implantable (316) avec une clé dynamométrique (610) pour ajuster l'élément de fixation (310 ; 402 ; 506).

9. Système selon la revendication 1, où la cloison flexible (302) contient de la silicone.

10. Système selon la revendication 1, où l'extrémité distale de l'élément de fixation (310 ; 402 ; 506) est sensiblement convexe (502 ; 508).

11. Système selon la revendication 1, où l'élément de fixation (310 ; 402 ; 506) comporte une cavité hexagonale prévue pour s'ajuster à l'outil (610).

12. Système selon la revendication 11, où la cavité hexagonale régulière comporte une fente (406).

13. Système selon la revendication 4, où le dispositif implantable (316) est un stimulateur cardiaque.

14. Système selon la revendication 4, où le dispositif implantable (316) est un défibrillateur interne à synchronisation automatique.

15. Système selon la revendication 2, comprenant en outre un lubrifiant appliqué sur la fente (308) de la cloison flexible (302).
